# EUROPEAN PATENT SPECIFICATION

(11) **EP 3 281 534 B1**
(45) Date of publication and mention of the grant of the patent: **08.05.2019**
(21) Application number: 16768721.9
(22) Date of filing: 18.03.2016
(51) Int. Cl.: A23L 27/00, A23L 2/00, A23L 2/60, A61K 8/34, A61K 8/60, A61Q 11/00, A23L 33/125, A23L 27/30, A23L 2/02, A23L 21/10, A23L 19/00

(54) **ERYTHRITOL-CONTAINING SWEETENER COMPOSITION**
ERYTHRITOLHALTIGE SÜSSSTOFFZUSAMMENSETZUNG
COMPOSITION ÉDULCORANTE CONTENANT DE L'ÉRYTHRITOL

(30) Priority: 20.03.2015 JP 2015058091
(43) Date of publication of application: 14.02.2018
(73) Proprietor: Matsutani Chemical Industry Co., Ltd., Itami-shi, Hyogo 664-8508 (JP)
(72) Inventor: FUJIHARA, Hideki, Itami-shi Hyogo 664-8508 (JP); TAKESHITA, Yumi, Itami-shi Hyogo 664-8508 (JP)
(74) Representative: Müller-Boré & Partner Patentanwälte PartG mbB
(86) International application number: PCT/JP2016/058875
(87) International publication number: WO 2016/152818

(56) References cited:
- EP-A1- 2 090 180
- EP-A2- 2 756 764
- WO-A1-2008/059623
- WO-A1-2011/139959
- WO-A1-2014/186084
- JP-A- 2013 524 851
- JP-A- 2014 526 258
- US-A1- 2014 322 389
- US-A1- 2014 342 044

## Description

### TECHNICAL FIELD

The present invention relates to a sweetener that contains erythritol as a main ingredient and is improved in quality of taste. The present invention also relates to an edible product or an oral care product containing the sweetener. The present invention further relates to a method for improving the quality of taste of a sweetener containing erythritol as a main ingredient.

### BACKGROUND ART

Sucrose is a sweetener excellent in quality of taste and is widely used as a main sweetener in foods and beverages. In recent years, however, it has been pointed out that excess intake of sucrose causes obesity and lifestyle-related diseases. Under such circumstances, low-calorie sweeteners as a sucrose alternative, which are intended for avoidance of obesity and lifestyle-related diseases, have been actively developed.

Sugar alcohols, especially erythritol, are used as a very low-calorie sweetener instead of sucrose in many foods and beverages. Erythritol, however, has disadvantages as compared to sucrose that it loses the taste too quickly after being taken, and that it leaves a peculiar bitter aftertaste. Consumers are familiar for years with the taste of so-called regular products containing sucrose or high fructose corn syrup, and they would not be satisfied with the quality of sweetness of erythritol alone. In addition, since the degree of sweetness of erythritol is about 70% that of sucrose, erythritol is used in a larger amount than sucrose when being used as an alternative sweetener to sucrose. Erythritol, however, has another disadvantage that it causes diarrhea when ingested in a large amount.

On the other hand, a high-intensity sweetener having a degree of sweetness several hundred times higher than that of sucrose can exhibit sweetness in a small amount, and is used for improving the quality of sweetness of erythritol based on its sweetness characteristics. For example, Patent Document 1 discloses that combination use of erythritol with acesulfame potassium and aspartame provides high sweetness intensity and a sucrose-like quality of taste. The technique of Patent Document 1, however, does not satisfy the preference of consumers who are familiar with the quality of taste of sucrose and high fructose corn syrup.

Among high-intensity sweeteners, stevia is naturally derived and is slow in rise of sweetness, and can complement the too quick loss of taste that is a disadvantage of erythritol. Thus, stevia is often used in combination with erythritol. Stevia, however, has peculiar bitterness similarly to erythritol. Accordingly, combination of stevia and erythritol still has the problem of bitterness and does not satisfy the preference of consumers.

Moreover, attempts have been made to improve the quality of sweetness by combining rare sugars which are slightly present in the natural world, such as D-psicose and D-tagatose, with erythritol (for example, Patent Documents 2 to 4).

Patent Document 2 discloses a method for improving the taste of a diet beverage including a step of adding at least one non-nutritive sweetener, a sugar alcohol, and D-tagatose to a diet beverage. That is, in Patent Document 2, the quality of taste of erythritol and a high-intensity sweetener is improved by the addition of D-tagatose to a sweetener containing a sugar alcohol and a high-intensity sweetener. Although D-tagatose has a degree of sweetness comparable to that of D-psicose, it has an energy of about 1.5 kilocalories, and is not a zero-calorie sweetener but a low-calorie sweetener. In addition, the taste quality-improving effect on erythritol exerted by the addition of D-tagatose is not necessarily sufficient, and the content of D-tagatose relative to erythritol should be increased for obtaining the effect. For example, in Patent Document 2, 0.1% or more of D-tagatose is added to a beverage containing erythritol and a high-intensity sweetener, and the content of D-tagatose is about twice that of erythritol. That is, Patent Document 2 does not disclose a technique for improving the quality of sweetness of a sweetener containing erythritol as a main ingredient.

Patent Document 3 discloses a D-psicose-containing low-calorie sweetener that is improved in quality of taste and that contains D-psicose as a main ingredient and also contains a sugar alcohol and/or a high-intensity sweetener. In Patent Document 3, a sugar alcohol and/or a high-intensity sweetener is used in combination with D-psicose in order to solve the problems such as the delay in the rise of sweetness of D-psicose and the heavy taste due to high-volume use of D-psicose. That is, Patent Document 3 employs a technique of limiting the amount of D-psicose when it is used as a main ingredient of a sweetener and complementing the insufficient sweetness with other sweeteners. Accordingly, Patent Document 3 does not disclose a technique for improving the quality of sweetness of a sweetener containing erythritol as a main ingredient.

Patent Document 4 discloses a composition containing D-psicose and erythritol together with at least one other edible ingredient, and foods and beverages containing the composition. Patent Document 4 merely discloses that the quality of taste of a food that requires sweetness is remarkably improved by using a high concentration of D-psicose that is almost at the same level with erythritol. Similarly to Patent Document 3, Patent Document 4 does not disclose a technique for improving the quality of sweetness of a sweetener containing erythritol as a main ingredient.

As described above, with respect to sweeteners containing erythritol as a main ingredient, a taste quality-improving technique for suppressing the bitterness and improving the aftertaste has been desired, but none of the conventional techniques is satisfactory.

### PRIOR ART DOCUMENTS

### PATENT DOCUMENTS

EP-A2-2756764 discloses a low-caloric sweetener with enhanced sweetening quality, comprising D-psicose, a sugar (alcohol) such as erythritol, but preferably tagatose, and optionally a high-intensity sweetener.
Patent Document 1: Japanese Patent Laid-Open Publication No. 2002-51723
Patent Document 2: Japanese Translation of PCT International Application Publication No. 2004-520072
Patent Document 3: Japanese Patent No. 4942001
Patent Document 4: U.S. Patent Application Publication No. 2014/0342044

### DISCLOSURE OF THE INVENTION

### PROBLEMS TO BE SOLVED BY THE INVENTION

An object of the present invention is to provide a sweetener composition that contains erythritol as a main ingredient and exhibits a good aftertaste with suppressed bitterness, and an edible product or an oral care product containing the sweetener composition. Another object of the present invention is to provide a sweetener composition that contains erythritol as a main ingredient and further contains a high-intensity sweetener (in particular, stevia), and exhibits a good aftertaste with suppressed bitterness, and an edible product or an oral care product containing the sweetener composition.

### MEANS FOR SOLVING THE PROBLEMS

The present inventors conducted diligent studies to solve the above-mentioned problems, and as a result, they found that a sweetener composition containing erythritol as a main ingredient and also containing a trace amount of D-psicose, that is, 0.27 to 7 parts by mass of D-psicose based on 100 parts by mass of erythritol, makes it possible to overcome the bitterness and bad aftertaste that are disadvantages of erythritol and dramatically improve the quality of taste. The present inventors also found that a sweetener composition containing erythritol as a main ingredient and further containing a high-intensity sweetener (in particular, stevia), and also containing a trace amount of D-psicose in the above-mentioned ratio makes it possible to suppress the bitterness which cannot be overcome by the conventional techniques and exhibit a good aftertaste.

### ADVANTAGES OF THE INVENTION

According to the present invention, in a sweetener composition containing erythritol as a main ingredient, it is possible to suppress the bitterness and give a good aftertaste, and improve the unpleasant quality of taste of erythritol. Further, in the present invention, improvement in the quality of taste of a sweetener composition containing erythritol as a main ingredient is realized by the addition of a trace amount of D-psicose which has less than a sweetness threshold concentration in an edible refined product. Thus, it is possible to improve the quality of taste of erythritol contained as a main ingredient without giving any influence of the quality of taste of psicose itself to the sweetener composition, and to provide a new non-calorie sweetener and a food or beverage containing the non-calorie sweetener.

Furthermore, according to the present invention, it is possible to suppress the bitterness and give a good aftertaste also in a sweetener composition containing erythritol as a main ingredient and further containing a high-intensity sweetener. Thus, it is possible to compensate for the disadvantage of the taste of erythritol and a high-intensity sweetener while taking advantage of the excellent sweetness characteristics of erythritol and the high-intensity sweetener, and to provide a non-calorie sweetener exhibiting an even more excellent taste and a food or beverage containing the sweetener.

### EMBODIMENTS OF THE INVENTION

### 1. Sweetener composition

The sweetener composition of the present invention contains erythritol as a main ingredient, and also contains 0.27 to 7 parts by mass of D-psicose based on 100 parts by mass of erythritol. Hereinafter, the sweetener composition of the present invention will be described in detail.

### (Erythritol)

The sweetener composition of the present invention contains erythritol as a main ingredient. In the present invention, the phrase "contain erythritol as a main ingredient" means that the percentage of erythritol is the highest among all the sweetener components contained in the sweetener composition of the present invention. More specifically, the phrase means that the amount of erythritol in 100 parts by mass in total of the sweetener components contained in the sweetener composition of the present invention is 90 parts by mass or more, preferably 95 to 99 parts by mass, more preferably 96 to 98 parts by mass.

The content of erythritol in the sweetener composition of the present invention is appropriately determined according to the form of the sweetener composition, and is, for example, 10 to 99% by mass. More specifically, when the sweetener composition of the present invention is in a liquid form, the content of erythritol is usually 20 to 99% by mass, preferably 50 to 95% by mass. When the sweetener composition of the present invention is in a solid form, the content of erythritol is usually 20 to 99% by mass, preferably 50 to 95% by mass.

### (D-psicose)

The sweetener composition of the present invention contains 0.3 to 3.4 parts by mass of D-psicose based on 100 parts by mass of erythritol contained as a main ingredient. Use of such a trace amount of D-psicose in combination with erythritol makes it possible to remedy the bitterness and bad aftertaste, which are disadvantages of erythritol, and to exhibit an excellent quality of taste.

As the D-psicose used in the present invention, any one of D-psicose purified from a natural product, D-psicose produced by an enzymological technique, D-psicose produced by chemical synthesis, and the like may be used. From the viewpoint of ease of production, D-psicose produced by an enzymological technique can be suitably used. In addition, D-psicose used in the present invention may be completely purified, or may contain a small amount of impurities.

As a method for producing D-psicose by an enzymological technique, for example, a method of epimerizing the 3-position of D-fructose using an epimerase (see, for example, Japanese Patent Laid-Open Publication No. 6-125776) can be mentioned. In addition, a syrupy D-psicose product can be obtained by epimerizing the 3-position of D-fructose through an enzymatic technique to produce D-psicose, and then optionally purifying the produced D-psicose by a method such as deproteinization, decolorization, or desalting, followed by concentration. Further, a purified product having a high purity of 99% or more can be obtained by subjecting the syrupy D-psicose product to column chromatography for fractionation and purification.

In the present invention, a commercially available product can also be used as the D-psicose. A commercially available product of D-psicose can be obtained, for example, from Wako Pure Chemical Industries, Ltd.

In the sweetener composition of the present invention, D-psicose is contained in an amount of 0.27 to 7 parts by mass based on 100 parts by mass of erythritol. Since the D-psicose content in the sweetener composition of the present invention is extremely small, when the sweetener composition of the present invention is added to an edible product or an oral care product so that the sweetener composition has a sucrose-converted sweetness concentration of 2 to 15% by mass, the concentration of D-psicose is less than the concentration at which D-psicose exhibit sweetness alone (sweetness threshold concentration). Such a trace amount of D-psicose, however, suppresses the bitterness of the sweetener composition containing erythritol as a main ingredient, and makes it possible to give a good aftertaste.

D-psicose can improve the quality of taste of erythritol in a trace amount of less than the sweetness threshold concentration as described above, whereas glucose does not exhibit such an effect. Fructose is an epimer of D-psicose and has a degree of sweetness about twice that of D-psicose, but can hardly improve the quality of taste of erythritol when used in a trace amount less than the sweetness threshold concentration. Tagatose is classified into rare sugars similarly to D-psicose, but can hardly improve the quality of taste of erythritol when used in a trace amount less than the sweetness threshold concentration as described above. That is, remedy of the bitterness and bad aftertaste of a sweetener composition containing erythritol as a main ingredient cannot be realized by the addition of other sweeteners at a concentration less than the sweetness threshold concentration, and it is important to select D-psicose and add D-psicose in a trace amount less than the sweetness threshold concentration.

From the viewpoint of more effectively suppressing the bitterness and realizing a good aftertaste with use of the sweetener composition of the present invention, the amount of D-psicose based on 100 parts by mass of erythritol is preferably 0.36 to 3.5 parts by mass, more preferably 0.5 to 3.0 parts by mass.

### (Additional sweeteners)

In addition to erythritol and D-psicose, the sweetener composition of the present invention may contain additional sweetener components as necessary.

The additional sweetener components that can be blended in the sweetener composition of the present invention are not particularly limited as long as they are commonly used as a sweetener in foods and beverages, oral medicines, oral compositions and the like. For example, high-intensity sweeteners, sugar, glucose, fructose, high fructose corn syrup, trehalose, tagatose, and sugar alcohols other than erythritol can be mentioned. These additional sweetener components may be used singly or in combination of two or more thereof.

Among these sweetener components, a high-intensity sweetener is preferable from the viewpoint of the taste quality-improving effect according to the present invention, consumers' attitude toward calorie control, and the like. The high-intensity sweetener may be either a naturally derived sweetener or an artificially synthesized sweetener. Examples of the naturally derived high-intensity sweetener include extracts of plants such as stevia, Luo Han Guo, and Thaumatococcus daniellii (family Marantaceae), and sweet components contained in these plant extracts. Specific examples of the sweet component contained in the stevia extract include stevioside and rebaudioside A. Specific examples of the sweet component contained in the Luo Han Guo extract include Mogroside V. Specific examples of the sweet component contained in the Thaumatococcus daniellii extract include thaumatin.

Examples of the artificial high-intensity sweetener include aspartame, acesulfame K, sucralose (registered trademark), saccharin, and neotame. These high-intensity sweeteners may be used singly or in combination of two or more thereof.

Among these high-intensity sweeteners, naturally derived sweeteners are preferable from the viewpoint of consumers' preference, and stevia and/or a sweet component thereof is especially preferable. Stevia and/or a sweet component thereof has an advantage that it can complement more effectively the low sustainability of the quality of sweetness, which is a disadvantage of erythritol, because it is slow in rise of sweetness, but has a disadvantage that it is bitter similarly to erythritol, and enhances the bitterness when used in combination with erythritol. In contrast, with use of the sweetener composition of the present invention, it is possible to sufficiently suppress the bitterness produced when erythritol and stevia and/or a sweet component thereof are used in combination, and consequently realize a remarkably excellent quality of taste.

When a high-intensity sweetener is blended in the sweetener composition of the present invention, the content thereof may be appropriately set according to the type of the high-intensity sweetener used. For example, the amount of the high-intensity sweetener is 0.2 parts by mass or more, preferably 0.5 to 2 parts by mass, more preferably 0.6 to 1 part by mass based on 100 parts by mass of erythritol.

### (Other components)

The sweetener composition of the present invention may contain, in addition to erythritol, D-psicose, and additional sweeteners added as necessary, various additives such as a taste component, an extender, an excipient, a diluent, a carrier, a perfume, a preservative, and an antioxidant as necessary.

### (Form and application)

The form of the sweetener composition of the present invention is not particularly limited, and the sweetener composition may be in a solid form such as a powder, a granule, and a tablet, or a liquid form such as an aqueous solution, an emulsion, and a suspension.

The sweetener composition of the present invention is added as a sweetener to an edible product or an oral care product requiring sweetness. The sweetener composition of the present invention may be used alone as a sweetener or may be used in combination with a conventionally used sweetener. For example, when the sweetener composition is used as an alternative to part of a sweetener having calories such as sucrose and fructose, it is possible to reduce the calorie, diversify the sweetness by the combination with other sweeteners, and satisfy the diversified preference of consumers.

The type of the edible product to which the sweetener composition of the present invention is added is not particularly limited as long as the product is ingested or taken orally. For example, foods and beverages and oral medicines can be mentioned.

Specific examples of foods and beverages to which the sweetener composition of the present invention is added include: seasonings such as soy sauce, powdered soy sauce, miso, powdered miso, moromi (fermented mash), hishio (fermented paste), sprinkles for rice, mayonnaise, dressing, vinegar, sanbaizu (vinegar, soy sauce and mirin mixed in roughly equal proportions), powdered sushi vinegar, Chinese soup, tempura sauce, noodle soup, sauce, ketchup, Korean barbecue sauce, curry roux, stew's ingredients, soup mix, Japanese-style broth, composite seasoning, mirin, mirin-like seasoning, table sugar, and coffee sugar; Japanese confectioneries such as rice cracker, cubic rice cracker, millet or rice cake, sticky rice cake, steamed bean-jam bun, uirou (sweet rice jelly paste), bean jam, adzuki-bean jelly, soft adzuki-bean jelly, Japanese agar jelly, jelly, castella sponge cake, and candy; Western confectioneries such as bread, biscuit, cracker, cookie, pie, pudding, butter cream, custard cream, cream puff, waffle, sponge cake, donut, chocolate, chewing gum, caramel, and candy; ice confectioneries such as ice cream and sherbet; syrups such as fruit syrup and shaved ice syrup; pastes such as flour paste, peanut paste, and fruit paste; fruit and vegetable processed foods such as jam, marmalade, fruit and vegetables pickled in syrup, and sugar confection; cereal processed foods such as bread, noodle, cooked rice, and artificial meat; pickles such as Fukujinzuke (sliced vegetables pickled in soy sauce), Bettarazuke (Japanese radish pickled in salted rice yeast), Senmaizuke (Japanese pickles of sliced turnip), and pickled Japanese scallions; seasonings for Japanese pickles such as those for Takuanzuke (yellow pickled radish) and Chinese cabbage pickles; meat products such as ham and sausage; fish meat products such as fish meat ham, fish meat sausage, kamaboko (boiled fish paste), chikuwa (fish paste shaped in a long tube), and tempura; delicacies such as sea urchin, salted squid guts, vinegared kelp, shredded dried cuttlefish, and grilled sun-dried puffer seasoned with mirin; Tsukudani (food boiled in soy sauce) made from seaweed, wild vegetables, dried cuttlefish, small fish, and shellfish; prepared foods such as cooked beans, potato salad, and simmered konbu rolls; bottled or canned products such as dairy products, fish meat, meat, fruit, and vegetables; alcoholic beverages such as synthetic wine, fruit wine, wines and spirits, and liquor; beverages such as coffee, cocoa, juice, carbonated drinks, sports drinks, fruit juice drinks, milk drinks, lactic acid beverages, lactic acid bacteria beverages, tea-based beverages, and nutritional drinks; premix powders such as pudding mixes and pancake mixes; instant foods and beverages such as instant juice, instant coffee, instant sweet red-bean soup powder, and instant soup; and supplements such as powders, capsules, tablets, and granules. Among these foods and beverages, beverages including carbonated drinks such as cola, and soft drinks such as sports drinks, fruit juice drinks, milk drinks, and tea-based beverages are required to be reduced in calorie, and are particularly suitable as a target to which the sweetener composition of the present invention is added.

Examples of oral medicines to which the sweetener composition of the present invention is added include sucrose-coated tablets. Sucrose-coated tablets are widely used as medicines such as solid preparations, because they mask the taste and smell of chemicals by their sucrose coating layers at the surface and thus are easy to take and excellent in appearance, and also have a moisture prevention effect. In general, a sucrose-coated tablet is produced by repeating the step of applying a sucrose solution to a tablet and drying the solution to complete the formation of a sucrose coating layer, adjusting the form of the tablet into an elliptical form, followed by glazing by the application of wax. A generally used sucrose solution for sucrose coating is a solution obtained by adding, to sucrose serving as a base, gelatin, gum arabic, polyvinyl pyrrolidone or the like as a binder to increase the strength of the sucrose coating layer of the sucrose-coated tablet. Powders such as talc, calcium sulfate, calcium phosphate, and precipitated calcium carbonate are also added as necessary.

Another example of an oral medicine to which the sweetener composition of the present invention is added is an internal medicine containing a bitter component such as barium. Adding the sweetener composition of the present invention to such a bitter internal medicine makes it possible to mask the bitterness by the sweetness and make the medicine easy to take.

Examples of oral care products to which the sweetener composition of the present invention is added include chewing gum, candy, tablet confectionery, film confectionery, toothpaste, liquid dentifrice, and mouthwash. As used herein, the term "oral care product" refers to a product that is chewed in the mouth or used for rinsing the mouth. The oral care products may also include products classified as a food or beverage or an oral medicine.

When the sweetener composition of the present invention is added to an edible product or an oral care product, the concentration of the composition is appropriately set according to the type of the product to which the composition is added and the degree of sweetness to be imparted to the product. Specifically, a concentration at which the composition can impart sweetness equivalent to 2 to 15% by weight in terms of sucrose-converted sweetness concentration to an edible product or an oral care product can be mentioned. As used herein, the term "sucrose-converted sweetness concentration" refers to the concentration of sucrose required to adjust the sweetness of a product to a level equal to the degree of sweetness imparted by the sweetener composition. In calculating the sucrose-converted sweetness concentration, the degree of sweetness of erythritol, D-psicose, and stevia relative to sucrose is 0.7, 0.7, and 350, respectively.

More specifically, the amount of the sweetener composition of the present invention added to the edible product or the oral care product is an amount at which the erythritol concentration in the edible product or the oral care product is 1 to 8% by mass, preferably 2 to 8% by mass, more preferably 2 to 7% by mass.

When the above-mentioned amount of the sweetener composition of the present invention is added to an edible product or an oral care product, erythritol has a concentration in the product at which it exhibits sweetness alone, but D-psicose has a concentration in the product less than or equal to the sweetness threshold concentration, at which it does not exhibit sweetness alone. In this manner, an interaction between erythritol having a concentration at which it exhibits sweetness alone and D-psicose having a concentration at which it does not exhibit sweetness alone makes it possible to impart a good aftertaste with suppressed bitterness and good sweetness to an edible product or an oral care product.

### 2. Method for improving quality of taste of sweetener containing erythritol as main ingredient

The taste quality-improving method of the present invention is a method for improving the quality of taste of a sweetener containing erythritol as a main ingredient, and includes adding, to the sweetener, 0.3 to 3.4 parts by mass of D-psicose based on 100 parts by mass of erythritol. In the taste quality-improving method of the present invention, the content of erythritol in the sweetener containing erythritol as a main ingredient, the types of additional sweeteners which may be contained as necessary in the sweetener containing erythritol as a main ingredient, a preferable content of D-psicose, and the form of the sweetener containing erythritol as a main ingredient are as described in "1. Sweetener composition" above.

In the taste quality-improving method of the present invention, when the sweetener containing erythritol as a main ingredient further contains stevia and/or a sweet component thereof, the combination of erythritol and stevia and/or a sweet component thereof can effectively suppress the produced bitterness. Thus, the taste quality-improving method can also be carried out as a method for suppressing the bitterness.

### EXAMPLES

Hereinafter, the present invention will be described in more detail with reference to reference examples and examples of the present invention.

### Reference Example 1: Measurement of sweetness threshold concentration of D-psicose

The sweetness threshold concentration of D-psicose was measured according to the method described in Bulletin of the Faculty of Education, Utsunomiya University, No. 63, Part 1 (March 2013). Specific test methods are as follows.

First, aqueous solutions having D-psicose concentrations gradually increased from 0.1% by mass to 1.0% by mass in 10 stages were prepared as test liquids. Subsequently, 9 trained panelists (designated as A to I in Table 1) were asked to taste the test liquids. They were asked to answer the lowest concentration at which they felt sweetness, and the average value of the concentrations was taken as the threshold of degree of sweetness.

The obtained results are shown in Table 1. From these results, it was found that the sweetness threshold concentration of D-psicose is about 0.5%.

**[Table 1]**

| Panelist | Lowest concentration at which panelist felt sweetness (% by mass) |
|---|---|
| A | 0.5 |
| B | 0.4 |
| C | 0.7 |
| D | 0.7 |
| E | 0.5 |
| F | 0.5 |
| G | 0.6 |
| H | 0.6 |
| I | 0.5 |
| Average | 0.55 |

### Example 1: Evaluation of taste quality-improving effect of D-psicose on erythritol (low concentration)

Part of a 15% by mass aqueous erythritol solution was substituted with D-psicose to prepare aqueous sweetener solutions having D-psicose concentrations gradually increased from 0% to 0.2%. In all of these aqueous sweetener solutions, the sucrose-converted sweetness concentration was unified to 10% by mass.

These aqueous sweetener solutions were ranked 0 to 5 in order from the lowest D-psicose concentration. The quality of taste of these aqueous sweetener solutions was evaluated by 9 trained panelists (designated as A to I in Table 3), and the rank of psicose concentration at which the taste quality-improving effect was exhibited was determined.

The compositions of the aqueous sweetener solutions, the rank of D-psicose concentration, and the evaluation results are shown in Table 2. The details of evaluation results of the panelists is shown in Table 3. As shown in Tables 2 and 3, it was found that addition of 0.27 parts by mass or more of D-psicose, based on 100 parts by mass of erythritol, to the aqueous sweetener solution suppressed the bitterness of erythritol, improved the aftertaste of the aqueous sweetener solution, and thus improved the quality of taste of the aqueous sweetener solution. In this case, the concentration of D-psicose was much lower than the sweetness threshold concentration of D-psicose (0.5% by mass).

**[Table 2]**

| Rank of D-psicose concentration | Numerical value in upper row: | | Taste quality-improving effect |
|---|---|---|---|
| | sweetener composition (% by mass) | | |
| | Numerical value in parentheses in lower row: amount based on 100 parts by mass of erythritol (parts by mass) | | |
| | Erythritol | Psicose | |
| 0 | 15 (100) | 0 (0.00) | No |
| 1 | 14.98 (100) | 0.02 (0.13) | No |
| 2 | 14.94 (100) | 0.06 (0.40) | Yes |
| 3 | 14.92 (100) | 0.08 (0.54) | Yes |
| 4 | 14.90 (100) | 0.10 (0.67) | Yes |
| 5 | 14.80 (100) | 0.20 (1.35) | Yes |

**[Table 3]**

| Panelist | Rank at which effect was exhibited | Panelists' comments |
|---|---|---|
| A | 2 | More crisp sweetness. |
| B | 2 | Refreshing and light feeling. |
| C | 1 | The aftertaste is improved. |
| D | 2 | The harsh aftertaste is masked. Number 5 is even more refreshing. |
| E | 2 | The aftertaste is refreshing. The harsh taste is lost. |
| F | 2 | The harsh taste is alleviated. |
| G | 2 | The sweetness is decreased. Number 5 is even more decreased in sweetness. |
| H | 2 | The rise of sweetness is weakened. |
| I | 1 | The aftertaste is sweeter. |

| | | |
|---|---|---|
| Average of rank at which effect was exhibited: 1.8 | | |

### Example 2: Evaluation of taste quality-improving effect of D-psicose on erythritol (high concentration)

Part of a 22% by mass aqueous erythritol solution was substituted with D-psicose to prepare aqueous sweetener solutions having D-psicose concentrations gradually increased from 0% to 0.2%. In all of these aqueous sweetener solutions, the sucrose-converted sweetness concentration was unified to 15% by mass.

These aqueous sweetener solutions were ranked 0 to 6 in order from the lowest D-psicose concentration. The quality of taste of these aqueous sweetener solutions was evaluated by 10 trained panelists (designated as A to J in Table 5), and the rank of psicose concentration at which the taste quality-improving effect was exhibited was determined.

The compositions of the aqueous sweetener solutions, the D-psicose concentration ranking, and the evaluation results are shown in Table 4. The details of evaluation results of the panelists is shown in Table 5. From these results, it was confirmed that even when the sucrose-converted sweetness concentration was increased to 15%, addition of 0.27 parts by mass or more of D-psicose, based on 100 parts by mass of erythritol, to the aqueous sweetener solution sufficiently suppressed the bitterness of erythritol, improved the aftertaste of the aqueous sweetener solution, and thus improved the quality of taste of the aqueous sweetener solution. Furthermore, it was also found from the panelists' comments that the quality of taste was better than in the case where the sucrose-converted sweetness concentration was 10% (Example 1). Also in this test in which high-concentration erythritol was used, the concentration of D-psicose, at which an improvement in the quality of taste was recognized, was much lower than the sweetness threshold concentration of D-psicose (0.5% by mass).

**[Table 4]**

| D-psicose concentration ranking | Numerical value in upper row: | | Taste quality-improving effect |
|---|---|---|---|
| | sweetener composition (% by mass) | | |
| | Numerical value in parentheses in lower row: amount based on 100 parts by mass of erythritol (parts by mass) | | |
| | Erythritol | Psicose | |
| 0 | 22 (100) | 0 (0.00) | No |
| 1 | 21.98 (100) | 0.02 (0.09) | No |
| 2 | 21.96 (100) | 0.04 (0.18) | Unclear |
| 3 | 21.94 (100) | 0.06 (0.27) | Yes |
| 4 | 21.92 (100) | 0.08 (0.36) | Yes |
| 5 | 21.90 (100) | 0.10 (0.46) | Yes |
| 6 | 21.80 (100) | 0.20 (0.92) | Yes |

**[Table 5]**

| Panelist | Rank at which effect was exhibited | Panelists' comments |
|---|---|---|
| A | 2 | The aftertaste of sweetness is refreshing. |
| B | 2 | The bitterness is lost. |
| C | 3 | The aftertaste is improved (the harsh taste is decreased). |
| D | 2 | The harsh aftertaste is masked. |
| E | 3 | The sweetness lasts. |
| F | 1 | The sweetness is more intense and lasts. |
| G | 2 | The aftertaste is improved. |
| H | 2 | The aftertaste is improved and the sweetness is mellowed. |
| I | 1 | The sweetness is mellowed. |
| J | 3 | The sweetness is refreshing. |

| | | |
|---|---|---|
| Average of rank at which effect was exhibited: 2.1 | | |

### Example 3: Evaluation of taste quality-improving effect of D-psicose on sweetener containing erythritol and stevia

Part of an aqueous solution containing 7.5% by mass of erythritol and 0.015% by mass of stevia was substituted with D-psicose to prepare aqueous sweetener solutions having D-psicose concentrations gradually increased from 0% to 0.2%. In all of these aqueous sweetener solutions, the sucrose-converted sweetness concentration was unified to 10% by mass.

These aqueous sweetener solutions were ranked 0 to 3 in order from the lowest D-psicose concentration. The quality of taste of these aqueous sweetener solutions was evaluated by 10 trained panelists (designated as A to J in Table 7), and the rank of psicose concentration at which the taste quality-improving effect was exhibited was determined.

The compositions of the aqueous sweetener solutions, the D-psicose concentration ranking, and the evaluation results are shown in Table 6. The details of evaluation results of the panelists is shown in Table 7. From the results in Tables 6 and 7, it was confirmed that even in the case of an aqueous erythritol solution containing stevia as a high-intensity sweetener, addition of 0.27 parts by mass or more of D-psicose, based on 100 parts by mass of erythritol, to the aqueous sweetener solution sufficiently suppresses the bitterness, and makes it possible to exhibit an excellent aftertaste. Also in this test, the concentration of D-psicose, at which an improvement in the quality of taste was recognized, was much lower than the sweetness threshold concentration of D-psicose (0.5% by mass).

**[Table 6]**

| D-psicose concentration ranking | Numerical value in upper row: composition of used sweetener composition (final concentration in beverage: % by mass) | | | Taste quality-improving effect |
|---|---|---|---|---|
| | Numerical value in parentheses in lower row: amount based on 100 parts by mass of erythritol (parts by mass) | | | |
| | Stevia | Erythritol | D-Psicose | |
| 0 | 0.015 (0.2) | 7.5 (100) | 0 (0.00) | No |
| 1 | 0.01492 (0.2) | 7.46 (100) | 0.08 (1.07) | Yes |
| 2 | 0.01490 (0.2) | 7.45 (100) | 0.10 (1.34) | Yes |
| 3 | 0.01480 (0.2) | 7.40 (100) | 0.20 (2.70) | Yes |

**[Table 7]**

| Panelist | Rank at which effect was exhibited | Panelists' comments |
|---|---|---|
| A | 3 | I feel no change. |
| B | 1 | No sweetness remains. The taste is mellowed. |
| C | 1 | The aftertaste is refreshing. |
| D | 2 | The aftertaste is refreshing. |
| E | 1 | The harsh aftertaste disappears and the solution has a refreshing feeling. |
| F | 2 | The sweetness is enriched. |
| G | 1 | The harsh taste disappears. |
| H | 1 | The aftertaste is improved. |
| I | 1 | The sweetness stands out. |
| J | 1 | No sweetness remains. |

| | | |
|---|---|---|
| Average of rank at which effect was exhibited: 1.4 | | |

### Example 4: Evaluation of taste quality-improving effect (taste exhibited in soft drink) of D-psicose on sweetener containing erythritol and stevia

A sweetener composition consisting of erythritol and stevia (erythritol: stevia weight ratio was 150 : 1) was prepared. Moreover, six sweetener compositions were prepared by substituting part of erythritol and stevia in the sweetener composition with D-psicose. These sweetener compositions were ranked 0 to 5 in order from the lowest D-psicose content.

A soft drink was prepared by adding each sweetener composition so as to have a sucrose-converted sweetness concentration of 10% by mass. Each soft drink contained 0.01% by mass of citric acid, 0.01% by mass of sodium citrate, and 0.1% by mass of perfume, and had concentrations of erythritol, stevia, and D-psicose as shown in Table 8. The quality of taste of these soft drinks was evaluated by 10 trained panelists (designated as A to J in Table 9), and the rank of psicose concentration at which the taste quality-improving effect was exhibited was determined.

The compositions of the soft drinks, the D-psicose concentration ranking in the sweetener compositions used, and the evaluation results are shown in Table 8. The details of evaluation results of the panelists is shown in Table 9. From the results in Tables 8 and 9, it was confirmed that addition of 0.27 to 7 parts by mass of D-psicose based on 100 parts by mass of erythritol to a beverage containing a sweetener composition, that contains stevia and erythritol, at a concentration of 10% by mass in terms of sucrose-converted sweetness concentration sufficiently suppresses the bitterness, and makes it possible to exhibit an excellent aftertaste. Also in this test, the concentration of D-psicose, at which an improvement in the quality of taste was recognized, was much lower than the sweetness threshold concentration of D-psicose (0.5% by mass).

**[Table 8]**

| D-psicose concentration ranking | Numerical value in upper row: composition of used sweetener composition (final concentration in beverage: % by mass) | | | Taste quality-improving effect |
|---|---|---|---|---|
| | Numerical value in parentheses in lower row: amount based on 100 parts by mass of erythritol (parts by mass) | | | |
| | Stevia | Erythritol | D-Psicose | |
| 0 | 0.02 (0.67) | 3.0 (100) | 0 (0.00) | No |
| 1 | 0.01996 (0.67) | 2.98 (100) | 0.04 (1.34) | Yes |
| 2 | 0.01994 (0.67) | 2.97 (100) | 0.06 (2.02) | Yes |
| 3 | 0.01992 (0.67) | 2.96 (100) | 0.08 (2.70) | Yes |
| 4 | 0.01990 (0.67) | 2.95 (100) | 0.10 (3.38) | Yes |
| 5 | 0.01980 (0.67) | 2.90 (100) | 0.20 (6.89) | Yes |

**[Table 9]**

| Panelist | Rank at which effect was exhibited | Panelists' comments |
|---|---|---|
| A | 1 | The bitterness is reduced. The sourness is emphasized. |
| B | 1 | The sourness is suppressed |
| C | 3 | The sweetness is refreshing. |
| D | 1 | The sourness is emphasized. |
| E | 1 | The aftertaste is refreshing. |
| F | 1 | The aftertaste is refreshing. |
| G | 1 | The sweetness is refreshing and the sourness is emphasized. |
| H | 2 | The sweetness and sourness are diminished. |
| I | 1 | The sourness is mellowed. |
| J | 1 | The aftertaste is refreshing. |

| | | |
|---|---|---|
| Average of rank at which effect was exhibited: 1.3 | | |

### Example 5: Evaluation of taste quality-improving effect on erythritol-containing jelly

A carbohydrate-free psicose-containing jelly that contained, as sweeteners, erythritol, stevia, and D-psicose at a concentration less than the sweetness threshold concentration and that had the composition shown in Table 10 was prepared. For comparison, a control jelly was prepared without adding D-psicose, and setting the sucrose-converted sweetness concentration to the value same as that of the psicose-containing jelly. For these jellies, a blind taste test by 10 panelists was conducted, and the panelists were asked to choose the one that was superior in quality of taste.

**[Table 10]**

| Raw materials | Control jelly (unit: g) | Psicose-containing jelly (unit: g) |
|---|---|---|
| Erythritol | 3.0 | 2.9 |
| D-Psicose | - | 0.1 |
| Stevia | 0.05 | 0.05 |
| Muscat juice | 0.5 | 0.5 |
| Citric acid | 0.2 | 0.2 |
| Vitamin C | 0.05 | 0.05 |
| Gelling agent | 1.4 | 1.4 |
| Flavoring agent | 0.2 | 0.2 |
| Water | Water is added to make the total amount of jelly 100 g | Water is added to make the total amount of jelly 100 g |

As a result, 2 panelists chose the control jelly, and 8 panelists chose the psicose-containing jelly. From these results, it was found that the quality of sweetness of a jelly is improved by adding to the jelly, as a sweetener composition, erythritol and stevia, and 0.27 to 7 parts by mass of D-psicose based on 100 parts by mass of erythritol.

### Example 6: Evaluation of taste quality-improving effect on erythritol-containing fruit sauce

A carbohydrate-free psicose-containing mango sauce that contained, as sweeteners, erythritol, stevia, and D-psicose at a concentration less than the sweetness threshold concentration and that had the composition shown in Table 11 was prepared. For comparison, a control mango sauce was prepared without adding D-psicose and setting the sucrose-converted sweetness concentration to the value same as that of the psicose-containing mango sauce. For these mango sauces, a blind taste test by 10 panelists was conducted, and the panelists were asked to choose the one that was superior in quality of taste.

**[Table 11]**

| Raw materials | Control mango sauce (unit: g) | Psicose-containing mango sauce (unit: g) |
|---|---|---|
| Erythritol | 6.0 | 5.9 |
| D-Psicose | - | 0.1 |
| Stevia | 0.03 | 0.03 |
| Mango pulp | 10.0 | 10.0 |
| Phosphoric acid-crosslinked starch | 3.0 | 3.0 |
| Hydroxypropylated phosphoric acid-crosslinked starch | 1.5 | 1.5 |
| Citric acid | 0.1 | 0.1 |
| Trisodium citrate | 0.2 | 0.2 |
| Perfume | 0.1 | 0.1 |
| Water | Water is added to make the total amount of mango sauce 100 g | Water is added to make the total amount of mango sauce 100 g |

As a result, 2 panelists chose the control mango sauce, and 8 panelists chose the psicose-containing mango sauce. From these results, it was found that the quality of sweetness of a mango sauce is improved by adding to the mango sauce, as a sweetener composition, erythritol and stevia, and 0.27 to 7 parts by mass of D-psicose (less than the sweetness threshold concentration) based on 100 parts by mass of erythritol.

### Example 7: Evaluation of taste quality-improving effect on erythritol-containing cola drink

A carbohydrate-free psicose-containing cola drink that contained, as sweeteners, erythritol, stevia, and D-psicose at a concentration less than the sweetness threshold concentration and that had the composition shown in Table 12 was prepared. For comparison, a control cola drink was prepared without adding D-psicose and setting the sucrose-converted sweetness concentration to the value same as that of the psicose-containing cola drink. For these cola drinks, a blind taste test by 10 panelists was conducted, and the panelists were asked to choose the one that was superior in quality of taste.

**[Table 12]**

| Raw materials | Control cola drink (unit: g) | Psicose-containing cola drink (unit: g) |
|---|---|---|
| Erythritol | 3.0 | 2.9 |
| D-Psicose | - | 0.1 |
| Stevia | 0.03 | 0.03 |
| O-phosphoric acid | 0.07 | 0.07 |
| Trisodium citrate | 0.04 | 0.04 |
| Caffeine | 0.01 | 0.01 |
| Caramel coloring | 0.10.28 | 0.10.28 |
| Perfume | 0.1 | 0.1 |
| Water, carbonated water | Water and carbonated water are added to make the total amount of cola drink 100 g | Water and carbonated water are added to make the total amount of cola drink 100 g |

As a result, one panelist chose the control cola drink, and 9 panelists chose the psicose-containing cola drink. From these results, it was found that the quality of sweetness of a cola drink is improved by adding to the cola drink, as a sweetener composition, erythritol and stevia, and 0.27 to 7 parts by mass of D-psicose (less than the sweetness threshold concentration) based on 100 parts by mass of erythritol.

### Example 8: Evaluation of taste quality-improving effect on erythritol-containing lime-flavored carbonated drink

A carbohydrate-free psicose-containing lime-flavored carbonated drink that contained, as sweeteners, erythritol, stevia, and psicose at a concentration less than the sweetness threshold concentration and that had the composition shown in Table 13 was prepared. For comparison, a control lime-flavored carbonated drink was prepared without adding D-psicose and setting the sucrose-converted sweetness concentration to the value same as that of the psicose-containing lime-flavored carbonated drink. For these lime-flavored carbonated drinks, a blind taste test by 10 panelists was conducted, and the panelists were asked to choose the one that was superior in quality of taste.

**[Table 13]**

| Raw materials | Control lime-flavored carbonated drink (unit: g) | Psicose-containing lime-flavored carbonated drink (unit: g) |
|---|---|---|
| Erythritol | 3.0 | 2.9 |
| D-Psicose | - | 0.1 |
| Stevia | 0.028 | 0.028 |
| Citric acid | 0.15 | 0.15 |
| Vitamin C | 0.02 | 0.02 |
| Potassium chloride | 0.02 | 0.02 |
| Perfume | 0.1 | 0.1 |
| Water, carbonated water | Water and carbonated water are added to make the total amount of lime-flavored carbonated drink 100 g | Water and carbonated water are added to make the total amount of lime-flavored carbonated drink 100 g |

As a result, 2 panelists chose the control lime-flavored carbonated drink, and 8 panelists chose the psicose-containing lime-flavored carbonated drink. From these results, it was found that the quality of sweetness of a lime-flavored carbonated drink is improved by adding to the lime-flavored carbonated drink, as a sweetener composition, erythritol and stevia, and 0.27 to 7 parts by mass of D-psicose (less than the sweetness threshold concentration) based on 100 parts by mass of erythritol.

### Example 9: Evaluation of taste quality-improving effect on erythritol-containing fruit juice drink

A carbohydrate-free psicose-containing fruit juice drink that contained, as sweeteners, erythritol, stevia, and psicose at a concentration less than the sweetness threshold concentration and that had the composition shown in Table 14 was prepared. For comparison, a control fruit juice drink was prepared without adding psicose and setting the sucrose-converted sweetness concentration to the value same as that of the psicose-containing fruit juice drink. For these fruit juice drinks, a blind taste test by 10 panelists was conducted, and the panelists were asked to choose the one that was superior in quality of taste.

**[Table 14]**

| Raw materials | Control fruit juice drink (unit: g) | Psicose-containing fruit juice drink (unit: g) |
|---|---|---|
| Erythritol | 3.0 | 2.9 |
| Psicose | - | 0.1 |
| Stevia | 0.02 | 0.02 |
| Concentrated orange juice | 2.0 | 2.0 |
| Concentrated lemon juice | 0.02 | 0.02 |
| Citric acid | 0.15 | 0.15 |
| Trisodium citrate | 0.02 | 0.02 |
| Vitamin C | 0.02 | 0.02 |
| Perfume | 0.15 | 0.15 |
| Water, carbonated water | Water and carbonated water are added to make the total amount of fruit juice drink 100 g | Water and carbonated water are added to make the total amount of fruit juice drink 100 g |

As a result, one panelist chose the control fruit juice drink, and 9 panelists chose the psicose-containing fruit juice drink. From these results, it was found that the quality of sweetness of a fruit juice drink is improved by adding to the fruit juice drink, as a sweetener composition, erythritol and stevia, and 0.27 to 7 parts by mass of D-psicose (less than the sweetness threshold concentration) based on 100 parts by mass of erythritol.

## Claims

1. A sweetener composition comprising erythritol as a main ingredient, and also comprising 0.27 to 7 parts by mass of D-psicose based on 100 parts by mass of erythritol.

2. The sweetener composition according to claim 1, further comprising a high-intensity sweetener.

3. The sweetener composition according to claim 2, wherein the high-intensity sweetener is stevia and/or a sweet component thereof.

4. The sweetener composition according to any one of claims 1 to 3, which is intended for being added to an edible product so that the edible product contains 1 to 8% by mass of erythritol.

5. An edible product comprising the sweetener composition according to any one of claims 1 to 4.

6. The edible product according to claim 5, which is a food or a beverage.

7. An oral care product comprising the sweetener composition according to any one of claims 1 to 4.

8. A method for improving quality of taste of a sweetener containing erythritol as a main ingredient, comprising adding, to a sweetener containing erythritol as a main ingredient, 0.3 to 3.4 parts by mass of D-psicose based on 100 parts by mass of erythritol.

## Patentansprüche

1. Süßstoffzusammensetzung, umfassend Erythrit als einen Hauptbestandteil und außerdem umfassend 0,27 bis 7 Massenteile von D-Psicose, bezogen auf 100 Massenteile von Erythrit.

2. Süßstoffzusammensetzung nach Anspruch 1, weiter umfassend einen Süßstoff mit hoher Intensität.

3. Süßstoffzusammensetzung nach Anspruch 2, wobei der Süßstoff mit hoher Intensität Stevia und/oder eine Süßkomponente davon ist.

4. Süßstoffzusammensetzung nach einem der Ansprüche 1 bis 3, welche dazu bestimmt ist, zu einem verzehrbaren Erzeugnis hinzugefügt zu werden, so dass das verzehrbare Erzeugnis 1 bis 8 Massen-% von Erythrit enthält.

5. Verzehrbares Erzeugnis, umfassend die Süßstoffzusammensetzung nach einem der Ansprüche 1 bis 4.

6. Verzehrbares Erzeugnis nach Anspruch 5, welches eine Speise oder ein Getränk ist.

7. Mundpflegeprodukt, umfassend die Süßstoffzusammensetzung nach einem der Ansprüche 1 bis 4.

8. Verfahren zur Verbesserung der Geschmacksqualität eines Süßstoffes, welcher Erythrit als einen Hauptbestandteil enthält, umfassend das Hinzufügen, zu einem Süßstoff, welcher Erythrit als einen Hauptbestandteil enthält, von 0,3 bis 3,4 Massenteilen von D-Psicose, bezogen auf 100 Massenteile von Erythrit.

## Revendications

1. Composition édulcorante comprenant de l'érythritol comme ingrédient principal, et comprenant également 0,27 à 7 parties en masse de D-psicose sur base de 100 parties en masse d'érythritol.

2. Composition édulcorante selon la revendication 1, comprenant en outre un édulcorant intense.

3. Composition édulcorante selon la revendication 2, dans laquelle l'édulcorant intense est du stévia et/ou un composant doux de celui-ci.

4. Composition édulcorante selon l'une quelconque des revendications 1 à 3, laquelle est destinée à être ajoutée à un produit comestible de sorte que ce produit comestible contient 1 à 8% en masse d'érythritol.

5. Produit comestible comprenant la composition édulcorante selon l'une quelconque des revendications 1 à 4.

6. Produit comestible selon la revendication 5, laquelle est un aliment ou une boisson.

7. Produit de soin buccal comprenant la composition édulcorante selon l'une quelconque des revendications 1 à 4.

8. Procédé d'amélioration de la qualité gustative d'un édulcorant contenant l'érythritol comme ingrédient principal, comprenant l'addition à un édulcorant contenant de l'érythritol comme ingrédient principal, de 0,3 à 3,4 parties en masse de D-psicose sur base de 100 parties en masse d'érythritol.
